# EUROPEAN PATENT APPLICATION

(11) **EP 2 942 011 A1**
(43) Date of publication of application: **11.11.2015**
(21) Application number: 13883143.3
(22) Date of filing: 26.06.2013
(51) Int. Cl.: A61B 5/12

(54) **DEVICE FOR HEARING TEST AND AUDITORY ASSESSMENT**

(30) Priority: 27.04.2013 CN 201310152215
(71) Applicant: Jiangsu Betterlife Medical Co., Ltd., Jiangsu 215500 (CN)
(72) Inventor: ZHAO, Bingbing, Jiangsu 215500 (CN); ZHAO, Yong David, Jiangsu 215500 (CN); ZHAO, Jennifer Jinping, Jiangsu 215500 (CN)
(74) Representative: Becker Kurig Straus
(86) International application number: PCT/CN2013/000758
(87) International publication number: WO 2014/172811

(57) **Abstract**

Disclosed is a device for hearing tests and auditory assessments, including: an all-digital audio and tone signal synthesis generator for converting a logic algorithm and a parameter into a digital signal via built-in software, and inputting the digital signal into a digital signal processor (DSP) or processing the same with a PC central processor (CPU) to generate and output a multi-modal audio and tone acoustic signal; a hearing test module, comprising one or more objective and/or subjective hearing test submodules, sharing corresponding stimulation or interference signals converted by signals input by the same all-digital audio and tone signal synthesis generator, in order to perform a hearing test on a patient; and a comprehensive auditory assessment module for performing comprehensive auditory assessment on the hearing test result output by each hearing test submodule.

## Description

### FIELD OF THE INVENTION

The present invention relates to the field of medical equipment, in particular to a device for hearing tests and auditory assessments.

### BACKGROUND OF THE INVENTION

Hearing test mainly checks the degree of hearing loss, which is a subjective or objective value, namely the decibels of a minimum sound that you can hear when harsh sounds are generated at difference frequencies; besides, values of air condition and bone conduction can also be used to judge deafness, namely neuropathic deafness, conductive deafness or a mixture of the former two. A hearing test is a method for diagnosing the hearing function state and auditory system diesels through the response of a tested patient to the sound stimulation.

A hearing test comprises an objective hearing test (for example, sound impedance OAE test, auditory brainstem response (ABR) test, auditory steady-state response (ASSR) test), and/or objective hearing test (for example, pure tone hearing test). Those hearing test devices that are different in principles and properties are mutually independent and are not mutually connected, employing self-prepared external single tone stimulating sound sources. However, whether the hearing level obtained by testing using those single tone stimulating sound sources can represent the sense level of the auditory nerve to various compound sound signals in real life is always doubted. Particularly, a deaf child can hear, but it does not mean that he/she can hear clearly and understand. In comparison with single, bald tones, some compound tones or speech that children are fond of can better stimulate their auditory nerves (for example, children are born to be very sensitive to some minimal sounds, music, and words like Mum and Papa), so the auditory level thereof can be more accurately reflected, the fitting and debugging of the hearing-aid be more effectively instructed, and the hearing-aid's effect of assisting hearing recovery be optimized. Therefore, it is very necessary to perform a comprehensive assessment on the hearing test results of various hearing test devices, expand the stimulating sound for testing from single tone to compound tones, and even simulated animal sound and human speech.

### SUMMARY OF THE INVENTION

The present invention mainly solves the technical problem of providing an integrated device for hearing test and auditory assessment and is simple to operate.

To solve the above technical problem the present invention employs a technical solution: a device for hearing tests and auditory assessments is provided, comprising:
an all-digital audio and tone signal synthesis generator for converting a logic algorithm and a parameter into a digital signal via built-in software by using the permutations of a plurality of standard tones and percussion instruments generated by the specifications of the internal standard for digital music/electronic synthesized music instruments " Digital interface MIDI of electronic music instruments", and inputting the digital signal into a digital signal processor (DSP) or processing same with a PC central processor (CPU) to generate and output a multi-modal audio and tone acoustic signal;
a hearing test module, comprising one or more objective and/or subjective hearing test submodules, sharing corresponding single tone and/or compound tone and/or simulated animal sounds and/simulated human voice stimulation signals converted by signals input by the same all-digital audio and tone signal synthesis generator, in order to perform a hearing test on a patient and output a hearing test result; and
a comprehensive auditory assessment module for performing comprehensive auditory assessment on a plurality of input hearing test results, in particular the assessment on the speech hearing capability.

In a preferable embodiment of the present invention, the all-digital audio and tone signal synthesis generator employs nonlinear, nonstable, dynamic and additive sound signal processing and analysis algorithm and logic algorithm, and the parameter varaibles of the algorithm include sound signal wave, time, amplitude, phase on condition of given frequency, while an analysis method employs time domain and/or spectra domain.

In a preferable embodiment of the present invention, the auditory assessment module comprises but is not limited to one or more objective and/or subjective hearing test sub-modules in pure tone hearing test, bone conduction bearing test, sound impedance OAE test, auditory brainstem response (ABR) test, auditory steady-state response (ASSR) test and speech test.

In a preferable embodiment of the present invention, one or more objective and/or subjective hearing test sub-modules share corresponding single tone and/or compound tone and/or simulated animal sounds and/simulated human voice stimulation signals converted by signals input by the same all-digital audio and tone signal synthesis generator, can randomly adjust the frequency and/or loudness and/or tone and/or melody and/or rhythm, generate the acoustic stimulation signals required by the hearing test, and perform objective and/or subjective hearing test.

In a preferable embodiment of the present invention, sound intensity adjusting modes comprise but are not limited to static manual adjustment, dynamic adding, constant sound intensity or gradual increase of the sound intensity, dynamic variation speed, intermittent or continuous sound.

In a preferable embodiment of the present invention, the output of the hearing test result is not limited to pure tone audiograms and similar hearing test charts, comprising a hearing threshold and an uncomfortable threshold; the pure tone audiograms are stored in the format of pictures or audio and tone signals to be output and printed or be in seamless connection with a hearing-aid fitting mode.

In a preferable embodiment of the present invention, the pure tone hearing test submodule also comprises a noise masking module and a noise environment selection module, provides use environment modes for selection of wearing of a hearing aid, and performs the hearing test in a simulated use environment.

In a preferable embodiment of the present invention, the device for hearing tests and auditory assessments also comprises an audio signal calibration module for perform single and/or more sound pressure level or sound intensity experimental calibration on the all-digital audio and tone signal synthesis generator at any given frequency.

In a preferable embodiment of the present invention, the device for hearing tests and auditory assessments also comprises an interaction module that a tested patient uses to operate the hearing test and auditory assessment through an external or internal user controller which comprises but is not limited to a Joystick; the tested patient can adjust the input variables comprising but not limited to the frequency, loudness, sound pressure level, noise mask, compound tone, simulated animal sound, simulated human voice, etc., perform the hearing test and auditory assessment and give the results to a professional operator for confirmation.

The present invention has the beneficial effects that: the device for hearing test and auditory assessment is a portable integrated comprehensive system, applicable to the objective and/or subjective hearing test and auditory assessment of groups from children to the old, is simple in operation, has an obvious effect, and can also obtain a relatively accurate hearing test and auditory assessment result through the operation of a patient.

### BRIEF DESCRIPTION OF THE DRAWINGS

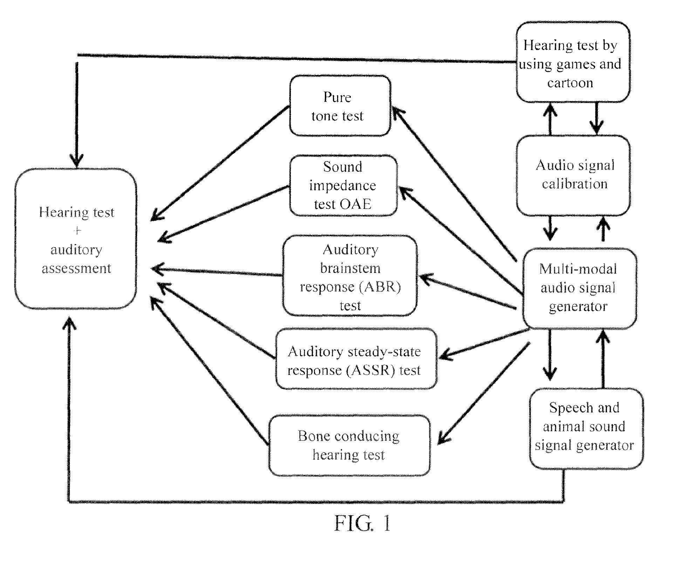

Figure 1 is a structure diagram of a device for a hearing test and auditory assessment in a preferable embodiment of the present invention.

### DETAILED DESCRIPTION OF THE INVENTION

The preferable embodiments are described in detail with reference to the attached drawings such that those skilled in this field can more easily understand the advantages and characteristics of the present invention to more clearly define the protective scope of the present invention.

Refer to figure 1, an embodiment of the present invention includes:

### A device for hearing test and auditory assessment.

The device for hearing test and auditory assessment comprises:
an all-digital audio and tone signal synthesis generator for converting a logic algorithm and a parameter into a digital signal via built-in software by using the permutations of a plurality of standard tones and percussion instruments generated by the specifications of the internal standard for digital music/electronic synthesized music instruments " Digital interface MIDI of electronic music instruments", and inputting the digital signal into a digital signal processor (DSP) or processing same with a PC central processor (CPU) to generate and output a multi-modal audio and tone acoustic signal;
a hearing test module, comprising one or more objective and/or subjective hearing test submodules, sharing corresponding single tone and/or compound tone and/or simulated animal sounds and/simulated human voice stimulation signals converted by signals input by the same all-digital audio and tone signal synthesis generator, in order to perform a hearing test on a patient and output a hearing test result;
and a comprehensive auditory assessment module for performing comprehensive auditory assessment on a plurality of hearing test results input.

### All-digital audio and tone signal synthesis generator:

The all-digital audio and tone signal synthesis generator complies a series of software and uses the permutations of 128 standard tone and 81 percussion instruments generated by the specifications of the internal standard for digital music/electronic synthesized music instruments " Digital interface MIDI of electronic music instruments" to convert the logic algorithm and parameters into digital signals and input the same into the digital signal processor (DSP) or processing same with a PC central processor (CPU) to generate and output a multi-modal audio and tone acoustic signal.

Nonlinear, nonstable, dynamic and additive sound signals are used to process the analysis algorithm and logic algorithm. Parameter variables of the algorithm comprise the sound signal waveform, time, amplitude, phase, etc. on condition of given frequency. The analysis method can employ a time domain or a spectra domain.

In a typical example, a dependent audio processing chip is used to reduce the occupation rate of the CPU resources, achieving better functions and audio effect.

In another typical example, a CODEC (digital-to-analogue signal conversion) chip is used to handle the conversion of the audio signals without occupying the CPU, ensuring the quality of the sound play and saving cost. The digital signals are converted into the analogue signal. The analogue signal is amplified, input into a loudspeaker and then output as a sound.

### Audio signal calibration module:

The audio signal calibration module performs sound pressure level or sound intensity experimental calibration at a given frequency on the entire audio software and hardware system of an integrated device including the specially configured loudspeaker earphones through a built-in or external hearing analyzer.

The sound calibration module can be operable to or automatically implement the sound pressure level or sound intensity experimental calibration within any set frequency and sound pressure level scope, for example, 150Hz - 8500Hz, 0dB - 120dB.

The loudspeaker earphone is placed in a sound cavity which simulates human ears to perform the air-conduction experimental calibration.

### Pure tone hearing test module:

The sound required by a hearing test is a pure tone signal of an intermittent or continuous single tone, including two variables, namely frequency and loudness.

For the hearing test with a set frequency, sound intensity adjusting modes comprise static manual adjustment, dynamic adding, constant sound intensity or gradual increase of the sound intensity, dynamic variation speed, intermittent or continuous sound. The dynamically added sound intensity scanning type hearing test can also be conveniently operated by a patient. The patient can operate the test by wearing the earphone, press the button or mouse, and quickly and accurately record the hearing threshold and uncomfortable threshold. The hearing test can be implemented in any set frequency and sound pressure level scope, for example, 150Hz - 8500Hz, 0dB - 120dB.

The output of the hearing test result is in the format of pure tone hearing diagram, including the hearing threshold and the uncomfortable threshold. The pure tone hearing diagram can be stored as picture document (pdf) and audio signal to be output and printed and be in seamless connection with the hearing-aid fitting module.

Direct heating test in the use environment of the patient wearing the hearing aid can avoid the problem that the pure tone hearing tested in the traditional mute room or sound isolating room environment cannot accurately reflect the nonlinear relationship of the hearing in the environment with noises; meanwhile, the hearing test and hearing-aid fitting integrated machine can seamlessly and directly output the test result to the fitting module, avoiding errors caused by the traditional manual input.

### Bone conduction hearing test module:

The bone conduction hearing test module stimulates the brain skin or skull through external sounds to conduct the hearing test. This hearing test is usually used as supplement of the hearing test, in particular when the air conduction obstacles are relatively large.

### Sound impedance test (OAE) module:

When a sound wave is transmitted within a media, on the one hand, the sound is blocked and resisted, which means sound impedance exists, and on the other hand, the sound is also conducted and received. The sum of the sound impedance and sound conduction and reception is called sound conduction impedance. The auris media of a person is an impedance matching device which can convert the acoustic energy into mechanical energy and amplify the same. Lesions such as hydrops in the auris media cavity and ossicular chain lesions or neoplasms on the sound conduction path change the impedance matching property and hinder the effective conduction of sound, thus conductive hearing loss is caused.

Diseases of the auris media are judged according to the dynamic characteristics of conduction by the auris media. An external sound stimulation is applied to test the audio energy which is generated at the cochlea, conducted by the ossicular chain and tympanic membrane and released to the external auditory canal or the change of the sound conduction and reception, which is particularly effective for the diagnosis of sensory deafness, auris media diseases and facial nerve diseases. Combining the pure tone hearing test, location, qualitative and quantitative diagnosis of the conductive hearing loss and mixed hearing loss can be implemented.

The response or sound pressure or tone, melody and rhythm are quickly changed at a given frequency according to the audio and tone signal generated by the all-digital audio and tone signal synthesis generator. The telephone receiver on the earphone generates a sound to vibrate the tympanic membrane so as to accurately control the multi-frequency scanning sound conduction and reception test of the tympanic membrane. This is a multi-frequency multi-component sound conduction and reception method, testing the sound reception and phase angle of the tympanic membrane plane on condition that the pressures on the two sides of the tympanic membrane are the same to seek the resonance frequency.

The pickup pressure sensor on the earphone probe detects the change of the sound pressure level before and after the resonance and inputs the same into a sound pressure balance meter, and the balance change curve reflects the change of the sound conduction and reception.

The built-in sound pressure balance meter automatically generates the balance change curve which reflects the change of the sound conduction and reception through the sound reception and phase angle data analysis and logistic algorithm treatment, thus judging the otitis media diseases.

### Auditory brainstem response (ABR) module:

The auditory brainstem response (ABR) module quickly changes the response, sound pressure, tone, melody and rhythm at a given frequency according to the audio and tone signal generated by the all-digital audio and tone signal synthesis generator, generates the potential (µV) change induced by response of the auditory system to a low-intensity short-tone (dB) stimulation, and compares the change of the potential waveform (mS-µV) recorded by the electrodes on the top of the head to judge the hearing loss, in particular, diagonalizing the nerve deafness and acoustic neuroma.

The evoked potential machine shares the pure tone hearing test module and the display module.

### Auditory steady-state response (ASSR) module:

ASSR is a response generated by stimulating the brain with continuous or steady sound simulating signals at a plurality of frequencies. ASSR is applied to objective hearing threshold evaluation and deafness diagnosis.

The all-digital audio and tone signal synthesis generator sends an audio signal, and the stimulating sound generated consists of two sine waves. One is high-frequency modulation wave, and the other is low-frequency modulation wave. A carrier wave is controlled through adjustment to the amplitude, frequency and phase of the modulation wave such that the characteristic parameters of the carrier wave vary with the modulation wave, and then the time domain signal is converted into the frequency spectra signal through fast fourier (FFT). The superimposed times of measurement and filtration scope are obtained through the signal analysis and logic algorithm.

With the stimulation of the high-frequency modulation wave, the auditory nerve system potential (µV) is induced to change, and the continuous response wave can be recorded at the cerebral cortex.

The low-impedance high-sensitivity electrode has a silver core externally wrapped by a multi-phase alloy MP35N composite electrode material that induces the potential machine to share the pure tone hearing test module and the display module. Technical requirements for recording are as follows: filter pass-band 10-300Hz (6dB/oct); amplifier gain 1x106, CMMR100-120dB, 16-bit A/D, and interelectrode resistance <5kΩ.

### Comprehensive auditory assessment module:

The comprehensive auditory assessment module integrating a plurality of hearing test methods stores the pure tone heating, bone conduction heating, sound impedance, auditory brainstem response, auditory steady-state response and speech test results in one file in formats of PDF picture documents and digital image documents.

Those documents can be output and printed, and can also be displayed on an integrated display for comparison and analysis.

Filling of the hearing diagnosis certificate is required, which can be output and printed in PDF document form and also stored in digital document form.

The device for hearing tests and auditory assessments also comprises an interaction module that a tested patient uses to operate the hearing test and auditory assessment through an external or internal user controller which comprises but is not limited to a Joystick; the tested patient can adjust the input variables comprising but that is not limited to the frequency, loudness, sound pressure level, noise mask, compound tone, simulated animal sound, simulated human voice, etc., perform the hearing test and auditory assessment and give the results to a professional operator for confirmation.

The above are only some embodiments of the present invention and shall not be regarded as limits to the present invention. Any equivalent structure or equivalent flow modifications made on the basis of the description and attached drawings of the present invention or director or indirect application to other related fields shall fall within the protective scope of the present invention.

## Claims

1. A device for hearing tests and auditory assessments, **characterized by** comprising:
an all-digital audio and tone signal synthesis generator for converting a logic algorithm and a parameter into a digital signal via built-in software by using the permutations of a plurality of standard tones and percussion instruments generated by the specifications of the internal standard for digital music/electronic synthesized music instruments "Digital interface MIDI of electronic music instruments", and inputting the digital signal into a digital signal processor (DSP) or processing same with a PC central processor (CPU) to generate and output a multi-modal audio and tone acoustic signal;
a hearing test module, comprising one or more objective and/or subjective hearing test submodules, sharing corresponding single tone and/or compound tone and/or simulated animal sounds and/simulated human voice stimulation signals converted by signals input by the same all-digital audio and tone signal synthesis generator, in order to perform an objective and/or subjective hearing test on a patient and output a hearing test result; and
a comprehensive auditory assessment module for performing comprehensive auditory assessment on a plurality of objective and/or subjective hearing test results input.

2. The device for hearing tests and auditory assessments according to claim 1, wherein the all-digital audio and tone signal synthesis generator employs nonlinear, nonstable, dynamic and additive sound signal processing and analysis algorithm and logic algorithm, and the parameter variables of the algorithm include, but are not limited to sound signal wave, time, amplitude, phase on condition of given frequency, while an analysis method employs time domain and/or spectra domain.

3. The device for hearing tests and auditory assessments according to claim 1, wherein the auditory assessment module comprises but is not limited to one or more objective and/or subjective hearing test sub-modules in pure tone hearing test, bone conduction bearing test, sound impedance OAE test, auditory brainstem response (ABR) test, auditory steady-state response (ASSR) test and speech test.

4. The device for hearing tests and auditory assessments according to claim 3, wherein said one or more objective and/or subjective hearing test sub-modules share corresponding single tone and/or compound tone and/or simulated animal sounds and/or simulated human voice stimulation signals converted by signals input by the same all-digital audio and tone signal synthesis generator, can randomly adjust the frequency and/or loudness and/or tone and/or melody and/or rhythm, generate the acoustic stimulation signals required by the hearing test, perform objective and/or subjective hearing test and comprehensive auditory assessment, in particular the assessment on the speech hearing capability.

5. The device for hearing tests and auditory assessments according to claim 4, wherein sound intensity adjusting modes comprise but are not limited to static manual adjustment, dynamic adding, constant sound intensity or gradual increase of the sound intensity, dynamic variation speed, intermittent or continuous sound.

6. The device for hearing tests and auditory assessments according to claim 4, wherein the output of the hearing test result is not limited to pure tone audiograms and similar hearing test charts, comprising a hearing threshold and an uncomfortable threshold; the pure tone audiograms are stored in the format of pictures or audio signals to be output and printed or be in seamless connection with a hearing-aid fitting mode.

7. The device for hearing tests and auditory assessments according to claim 4, wherein the pure tone hearing test submodule also comprises a noise masking module and a noise environment selection module, provides use environment modes for the selection of wearing of a hearing aid, and performs the hearing test in a simulated use environment.

8. The device for hearing tests and auditory assessments according to claim 1, wherein the device for hearing tests and auditory assessments also comprises an audio signal calibration module for perform single and/or more sound pressure level or sound intensity experimental calibration on the all-digital audio and tone signal synthesis generator at any given frequency.

9. The device for hearing tests and auditory assessments according to claim 1, wherein the device for hearing tests and auditory assessments also comprises an interaction module that a tested patient uses to operate the hearing test and auditory assessment through an external or internal user controller which comprises but is not limited to a Joystick; the tested patient can adjust the input variables comprising but not limited to the frequency, loudness, sound pressure level, noise mask, compound tone, simulated animal sound, simulated human voice, etc., perform the hearing test and auditory assessment and give the results to a professional operator for confirmation.
